# EUROPÄISCHE PATENTSCHRIFT

(11) **EP 1 191 106 B1**
(45) Veröffentlichungstag und Bekanntmachung des Hinweises auf die Patenterteilung: **28.04.2004**
(21) Anmeldenummer: 01120750.3
(22) Anmeldetag: 06.09.2001
(51) Int. Cl.: C12P 13/04, C12P 13/06, C12P 13/12, C07D 257/04, C07D 249/04, C07D 249/08, C07D 249/18, C07D 275/02, C07D 275/04, C07D 333/06, C07D 233/61, C07D 213/04, C07D 261/20, C07D 239/26

(54) **Verfahren zur fermentativen Herstellung von nicht-proteinogenen L-Aminosäuren**
Process for the fermentative preparation of non-proteinogenic L-amino acids
Procédé de préparation par fermentation d'acides L-aminiques non protéinogènes

(30) Priorität: 21.09.2000 DE 10046934
(43) Veröffentlichungstag der Anmeldung: 27.03.2002
(73) Patentinhaber: Consortium für elektrochemische Industrie GmbH, 81379 München (DE)
(72) Erfinder: Maier, Thomas, Dr., 85221 Dachau (DE)
(74) Vertreter: Potten, Holger

(56) Entgegenhaltungen:
- WO-A-97/15673
- US-A- 5 646 167
- US-A- 5 972 663
- SAITO ET AL: "production of plant non-protein amino acids by recombinant enzymes of sequential biosynthetic reactions in bacteria" BIOLOGICAL AND PHARMACEUTICAL BULLETIN, TOKYO, JP, Bd. 20, Nr. 1, Januar 1997 (1997-01), Seiten 47-53, XP002122799 ISSN: 0918-6158
- DENK D ET AL: "L-CYSTEINE BIOSYNTHESIS IN ESCHERICHIA COLI: NUCLEOTIDE SEQUENCE AND EXPRESSION OF THE SERINE ACETYLTRANSFERASE (CYSE) GENE FROM THE WILD-TYPE AND A CYSTEINE-EXCRETING MUTANT" JOURNAL OF GENERAL MICROBIOLOGY, SOCIETY FOR MICROBIOLOGY, READING, GB, Bd. 133, Nr. 3, 1. März 1987 (1987-03-01), Seiten 515-525, XP000617605 ISSN: 0022-1287
- NAKAMORI ET AL: "overproduction of L-cysteine and L-cystine by Escherichia coli strains with a genetically altered serine acetyltransferase" APPLIED AND ENVIRONMENTAL MICROBIOLOGY, WASHINGTON,DC, US, Bd. 64, Nr. 5, Mai 1998 (1998-05), Seiten 1607-1611, XP002115630 ISSN: 0099-2240
- TOPCZEWSKI ET AL: "Cloning and characterization of the Aspergillus nidulans cysB gene encoding cysteine synthase" CURRENT GENETICS, NEW YORK, NY, US, Bd. 31, Nr. 4, April 1997 (1997-04), Seiten 348-356, XP002109934 ISSN: 0172-8083
- DATABASE CA [Online] CHEMICAL ABSTRACTS SERVICE, COLUMBUS, OHIO, US; GASKIN, PETER J ET AL: "The C-S lysis of l-cysteine conjugates by aspartate and alanine aminotransferase enzymes" retrieved from STN Database accession no. 123:163090 XP002182677 & HUM. EXP. TOXICOL. Bd. 14, Nr. 5, 1995, Seiten 422 - 427
- DATABASE CA [Online] CHEMICAL ABSTRACTS SERVICE, COLUMBUS, OHIO, US; TAL, ABRAHAM ET AL: "Glutathione conjugation: a detoxification pathway for fenoxaprop-ethyl in barley, crabgrass, oat, and wheat" retrieved from STN Database accession no. 120:99262 XP002182678 & PESTIC. BIOCHEM. PHYSIOL. Bd. 16, Nr. 3, 1993, Seiten 190 - 199
- DATABASE CA [Online] CHEMICAL ABSTRACTS SERVICE, COLUMBUS, OHIO, US; GRZONKA, Z. ET AL: "Tetrazole analogs of amino acids as a tool in studies of the role of free carboxyl group in biologically active systems" retrieved from STN Database accession no. 88:165656 XP002182679 & GOODMAN M.; MEIENHOFER J.: 'PROC. AM. PEPT, SYMP., 5TH', 1997, WILEY, NEW YORK
- DATABASE CA [Online] CHEMICAL ABSTRACTS SERVICE, COLUMBUS, OHIO, US; PAL, BIMAL C. ET AL: "Reaction of 5-halocytosine derivatives with cysteine" retrieved from STN Database accession no. 109:190731 XP002182680 & NUCLEOSIDES NUCLEOTIDES Bd. 7, Nr. 1, 1988, Seiten 1 - 21
- DATABASE CA [Online] CHEMICAL ABSTRACTS SERVICE, COLUMBUS, OHIO, US; VOTRUBA, IVAN ET AL: "Conversion of 2-mercaptopyrimidine into S-(pyrimidin-2-yl)cysteine in growing Escherichia coli cells" retrieved from STN Database accession no. 77:70746 XP002182681 & FEBS (FED. EUR. BIOCHEM. SOC.) LETT. Bd. 22, Nr. 3, 1972, Seiten 287 - 288
- DATABASE CA [Online] CHEMICAL ABSTRACTS SERVICE, COLUMBUS, OHIO, US; WISLOCKI, PETER G. ET AL: "Drug residue formation from ronidazole, a 5-nitroimidazole. VI. Lack of mutagenic activity of reduced metabolites and derivatives of ronidazole" retrieved from STN Database accession no. 101:71202 XP002182683 & CHEM.-BIOL. INTERACT. Bd. 49, Nr. 1-2, 1984, Seiten 27 - 38
- DATABASE CA [Online] CHEMICAL ABSTRACTS SERVICE, COLUMBUS, OHIO, US; GIRARD, MICHEL ET AL: "5-Nitroimidazoles. II. Unexpected reactivity of ronidazole and dimetridazole with thiols" retrieved from STN Database accession no. 120:271118 XP002182684 & CAN. J. CHEM. Bd. 71, Nr. 9, 1993, Seiten 1349 - 1352
- DATABASE CA [Online] CHEMICAL ABSTRACTS SERVICE, COLUMBUS, OHIO, US; SAGIYAN, A. S. ET AL: "Novel approach to asymmetric synthesis of non-proteinogenic heterocyclic L-.alpha.-amino acids" retrieved from STN Database accession no. 135:19890 XP002182686 & KHIM. ZH. ARM. Bd. 53, Nr. 3-4, 2000, Seiten 118 - 120
- DATABASE STN FILE REG [Online] 1997 Database accession no. 193630-66-9
- DATABASE STN FILE REG [Online] Database accession no. 247904-45-6
- DATABASE STN FILE REG [Online] 2000 Database accession no. 342047-43-2
- DATABASE STN FILE REG [Online] 1978 Database accession no. 38764-16-8

## Beschreibung

Die Erfindung betrifft Verfahren zur Herstellung von nicht-proteinogenen L-Aminosäuren durch direkte Fermentation von Mikroorganismen sowie nach dem Verfahren erhaltene L-Aminosäuren.

Nicht-proteinogene Aminosäuren sind Aminosäuren, die in der Natur nicht als Bausteine für die Proteinbiosynthese verwendet werden und dadurch klar von den 20 proteinogenen Aminosäuren abzugrenzen sind. Es handelt sich vorzugsweise um β-substituierte L-Alanin-Derivate.

Nicht-proteinogene Aminosäuren stellen interessante Verbindungen z.B. für die Herstellung von Pharma- und Agrowirkstoffen dar. Sie können als Wirkstoff oder als Teil eines Wirkstoffs in einer Art molekularer Mimikry die Struktur von natürlichen Aminosäuren imitieren und dadurch zum Beispiel bei Rezeptor-Interaktionen eine Modulation der natürlichen Reaktion bewirken. Zudem können sie ganz allgemein als chirale Verbindungen im Rahmen des "chiral pool" als Synthesebausteine dienen.

Bisherige Herstellverfahren für nicht-proteinogene Aminosäuren in enantiomerenreiner Form basieren meist auf aufwendigen Synthesen, die zudem meist nur Zugang zu einer bestimmten Verbindung erlauben. Nur wenige Verfahren ermöglichen durch den einfachen Austausch eines Eduktes die Herstellung verschiedener Verbindungen.

In den meisten Fällen handelt es sich um chemische Synthesen, die ihrerseits meist schon von chiralen Bausteinen ausgehen oder eine Racematspaltung nach sich ziehen.

Alternativ sind auch einige enzymatische Verfahren beschrieben. So können mit Hilfe von Transaminasen aus α-Ketosäuren mit L-Glutaminsäure als Amino-Donor verschiedene nicht-proteinogene Aminosäuren hergestellt werden. Ein anderes Verfahren benützt Hydantoinasen in Kombination mit Carbamoylasen. Enzymatische Verfahren sind jedoch ebenfalls kostenintensiv, da die entsprechenden Enzyme bereitgestellt werden müssen und diese als Katalysatoren nur begrenzte Lebenszeit aufweisen (Rehm et al. Biotechnology 1996; Vol.6 S. 505 - 560).

Besonders einfach und günstig wären dagegen Verfahren zur Herstellung von nicht-proteinogenen Aminosäuren durch direkte Fermentation von Mikroorganismen. Solche Verfahren bergen jedoch die Gefahr, dass die produzierte nicht-proteinogene Aminosäure mit dem Stoffwechsel der natürlichen Aminosäuren interferiert und es damit zu Wachstumshemmungen kommt. Bisher ist in diesem Themenbereich ein Verfahren zur direkten Fermentation von D-Aminosäuren bekannt (W098/14602). Diese Anmeldung beschreibt die Herstellung von D-Aminosäuren mittels rekombinanter Mikroorganismen, in die ein D-Aminotransferase Gen und ein L-Aminodeaminase Gen eingebracht wurde. Darüberhinaus beschrieben Saito et al. (Biol. Pharm. Bull. 1997, 20: 47-53) die Produktion der pflanzlichen, nicht-proteinogenen Aminosäure L-Pyrazolylalanin durch Expression von pflanzlichen Genen in Escherichia coli. Die Ausbeuten sind für eine kommerzielle Produktion mit < 1 g/l jedoch zu niedrig und die Kosten beim beschriebenen Einsatz von L-Serin als Edukt sehr hoch.

Die Dokumente Denk D., et al.: "L-Cysteine Biosynthesis in Escherichia Coli: Nucleotide Sequence and Expression of the Serine Acetyltransferase (CYSE) Gene from the Wild-Type and a Cysteine-Excreting Mutant", Journal of General Microbiology, Society for Microbiology, Reading, GB, vol. 133, no. 3, March 1, 1987, pages 515 - 525, Nakamori, et al.: "Overproduction of L-cysteine and L-cystine by Escherichia coli strains with a genetically altered serine acetyltransferase", Applied and Environmental Microbiology, Washington, DC, US, vol. 64, no. 5, May 5, 1998, pages 1607 - 1611 and Topczewski, et al.: "Cloning and characterization of the Aspergillus nidulans cysB gene encoding cysteine synthase" Current Genetics, New York, NY, US, vol 31, no. 4, April, 1997, pages 348 - 356 beschreiben Mikroorganismen mit einem deregulierten Cysteinstoffwechsel, die zur Herstellung natürlicher Aminosäuren Verwendung finden.

Aufgabe der vorliegenden Erfindung ist es, ein effizientes Verfahren zur Herstellung einer Reihe von nicht-proteinogenen L-Aminosäuren durch direkte Fermentation zur Verfügung zu stellen.

Diese Aufgabe wird dadurch gelöst, daß ein Mikroorganismenstamm mit einem modifizierten cys-E-Allel fermentiert wird, dadurch gekennzeichnet, daß während der Fermentation eine nukleophile Verbindung in derartigen Mengen dem Fermentationsansatz zudosiert wird, daß diese zur Produktion von nicht-proteinogenen L-Aminosäuren durch den Mikroorganismenstamm führt.

Vorzugsweise werden am Ende der Fermentation die nicht-proteinogenen L-Aminosäuren aus dem jeweiligen Fermentationsansatz mittels an sich bekannter Methoden abgetrennt.

Überraschenderweise wurde gefunden, daß bei der Fermentation von Mikroorganismenstämmen mit dereguliertem Cystein-Stoffwechsel anstelle von Sulfid ein Reihe anderer nukleophiler Verbindungen sehr effizient in den Aminosäurestoffwechsel eingehen und die entsprechenden Reaktionsprodukte ins Kulturmedium sezerniert werden. Vorteilhafterweise kann dabei Glucose als billige Kohlenstoffquelle verwendet werden.

Durch die erfindungsgemäße Zudosierung von nukleophilen Verbindungen während der Fermentation werden demnach nicht-proteinogene L-Aminosäuren gebildet. Vorzugsweise wird daher eine nukleophile Verbindung, die in den Aminosäurestoffwechsel eingeht, während der Fermentation zugegeben.

Bevorzugt werden nukleophile Verbindungen zugegeben, die einen Rest ausgewählt aus der Gruppe 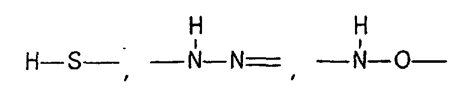 umfassen.

Besonders bevorzugt wird dem Fermentationsansatz eine nukleophile Verbindung ausgewählt aus der folgenden Gruppe zugegeben:
- Thiol der allgemeinen Formel (1):

   H―S―R₁ (1)

   wobei R¹ einwertiger substituierter oder nicht substituierter Alkyl-, Alkoxy-, Aryl- oder Heteroarylrest mit maximal 15 C-Atomen bedeutet;

- Azol der allgemeinen Formel (2) oder (3): 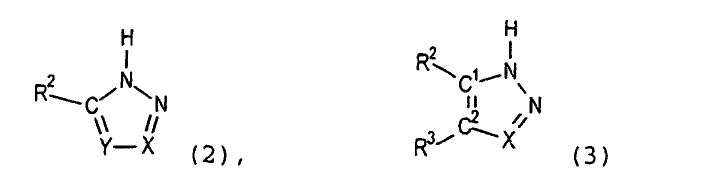 sowie deren Ester, Ether oder Salze,
   wobei X und Y gleich oder verschieden sind und CR⁴ oder N bedeuten und R⁴ -H, -COOH, -OH, -NH₂, -NO₂⁻, -SH, -SO₃⁻, -F, -Cl, -Br, -I, C₁-C₅-Alkylcarbonyl- oder R¹ bedeutet und R¹ die zu Formel (1) genannte Bedeutung hat und
   wobei R² und R³ gleich oder verschieden sind und R⁴ bedeuten oder wobei C¹ und C² in Formel (3) anstelle der Substituenten R² und R³ mittels einer Brücke [-CR⁵R⁶-]ₐ mit a gleich 1, 2, 3 oder 4 zu einem Ring verknüpft sind, wobei
   R⁵ und R⁶ gleich oder verschieden sind und R⁴ bedeuten und eine oder mehrere nicht benachbarte Gruppen [-CR⁵R⁶-] durch Sauerstoff, Schwefel oder einen ggf. mit C₁-C₅-Alkyl- substituierten Iminorest ersetzt sein können und
   zwei benachbarte Gruppen [-CR⁵R⁶-] durch eine Gruppe [-CR⁵=CR⁶-] oder durch eine Gruppe [-CR⁵=N-] ersetzt sein können.
- Isoxazolinon der allgemeinen Formel (4) oder (5): 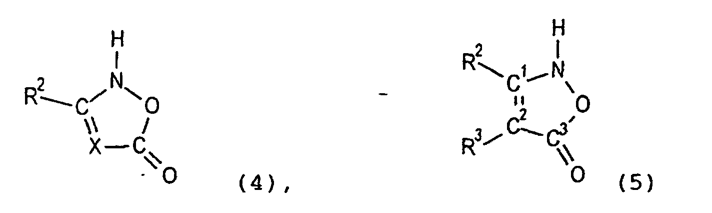 sowie deren Ester, Ether oder Salze,
   wobei X, R¹ , R² und R³ die bereits genannte Bedeutung haben und
   wobei C¹ und C² in Formel (5) anstelle der Substituenten R² und R³ mittels einer wie für Formel (3) definierten Brücke zu einem Ring verknüpft sein kann.

Beispiele für Thiole sind Verbindungen ausgewählt aus der Gruppe 2-Mercaptoethanol, 3-Mercaptopropanol, 3-Mercaptopropionsäure, 3-Mercapto-1-propansulfonsäure, Mercaptoethansulfonsäure, 2-Mercaptoethylamin, Thioglycolsäure, Thiomilchsäure, Thioessigsäure, Mercaptobernsteinsäure, Mercaptobrenztraubensäure, Dithiothreitol, Dithioerythritol, 1-Thioglycerin, Thiophenol, 4- Fluorthiophenol, 4-Mercaptophenol, p-Thiokresol, 5-Thio-2-nitrobenzoesäure, 2-Mercaptothiazol, 2-Mercaptothiazolin, 2-Mercaptoimidazol, 3-Mercapto-1,2,4-Triazol, 2-Thiophenthiol, 2-Mercaptopyridin, 2-Mercaptopyrimidin, 2-Thiocytosin, 2-Mercaptonicotinsäure,2-Mercapto-1-methylimidazol, 2-Mercaptobenzothiazol, 2-Mercaptobenzoxazol, 6-Mercaptopurin.

Beispiele für Azole sind Verbindungen ausgewählt aus der Gruppe 1,2-Pyrazol, 3-Methylpyrazol, 4-Methylpyrazol, 3,5-Dimethylpyrazol, 3-Aminopyrazol, 4-Aminopyrazol, Pyrazol-4-carbonsäure, Pyrazol-3,5-dicarbonsäure, 1,2,3-Triazol, 1,2,4-Triazol, 3-Amino-1,2,4-Triazol, 1,2,3,4-Tetrazol, Indazol, Indazol-3-carbonsäure, Indazol-5-carbonsäure, 5-Aminoindazol, Benzotriazol, Benzotriazol-5-carbonsäure, 5-Aminobenzotriazol, Aminopyrazolopyrimidin, 8-Azaguanin, 8-Azaadenin.

Beispiele für Isoxazolinone sind Verbindungen ausgewählt aus der Gruppe Isoxazolin-2-on, 4-Methylisoxazolin-2-on, 5-Methylisoxazolin-2-on, 4,5-Dimethylisoxazolin-2-on, 1,2,4-Oxadiazolidin-3,5-dion.

Mikroorganismenstämme mit dereguliertem Cystein-Stoffwechsel, die im erfindungsgemäßen Verfahren eingesetzt werden können, sind aus dem Stand der Technik bekannt. Sie zeichnen sich durch eine gegenüber dem Wildtyp-Stamm erhöhte endogene Produktion von O-Acetyl-L-Serin - dem unmittelbaren biosynthetischen Vorläufer von L-Cystein - aus. In einem Mikroorganismus wird im letzten Schritt der Cysteinbiosynthese bekanntermaßen durch die Aktivität der O-Acetyl-Serin-Sulfhydrylasen die Acetyl-Funktion des O-Acetyl-L-Serins gegen eine Thiol-Funktion ersetzt und damit L-Cystein gebildet. Dieser Reaktionstyp wird als β-Substitution bezeichnet, da am β-Kohlenstoffatom der Aminosäure ein Austausch einer funktionellen Gruppe vorgenommen wird.

Vorzugsweise wird einer der folgenden Mikroorganismenstämme im erfindungsgemäßen Verfahren eingesetzt:
- Stämme mit modifizierten cysE-Allelen wie beispielsweise in WO 97/15673 oder Nakamori S. et al., 1998, Appl. Env. Microbiol. 64: 1607-1611 oder Takagi H. et al., 1999, FEBS Lett. 452: 323-327 beschrieben, oder
- Stämme, die Efflux-Gene enthalten, wie sie beispielsweise in EP 0885962 A1 beschreiben sind, oder
- Stämme mit einer geänderten CysB-Aktivität wie in der deutschen Patentanmeldung DE 19949579 beschrieben, oder
- Stämme, die unter Einsatz unspezifischer Mutagenese-Methoden kombiniert mit Screening-Methoden für Cystein-Überproduktion oder verminderten Cystein-Abbau gewonnen werden, wie beispielsweise in WO 97/15673 oder in Nakamori S. et al., 1998, Appl. Env. Microbiol. 64:1607-1611 beschrieben.

Solche Stämme zeichnen sich dadurch aus, daß sie bei ausreichender Zuführung einer anorganischen Schwefel-Quelle wie z.B. Sulfat oder Thiosulfat signifikante Mengen an L-Cystein oder eines Derivates davon in das Kulturmedium sezernieren. Durch die erfindungsgemäße Zudosierung einer nukleophilen Verbindunge während der Fermentation geht diese Verbindung in die β-Substitution ein und führt dadurch zur Produktion von nicht-proteinogenen L-Aminosäuren.

Mit Mikroorganismenstämmen, die keinen deregulierten Cystein-Stoffwechsel besitzen (z.B. den üblichen Wildtyp-Organismen) führt ein derartiges Vorgehen zum Erlahmen der Cysteinbiosynthese und damit zu einer Wachstumshemmung. Es werden demzufolge keine nicht-proteinogenen Aminosäuren in signifikanten Mengen gebildet.

Da die erfindungsgemäß verwendeten Stämme aber einen deregulierten Cystein-Stoffwechsel und damit einen hohen endogenen Spiegel an O-Acetyl-L-Serin aufweisen, ist die Produktion der nicht-proteinogenen L-Aminosäure in großen Mengen möglich. Gleichzeitig ist noch eine ausreichende Bildung von L-Cystein gewährleistet, um das Zellwachstum des Mikroorganismus zu garantieren.

Bevorzugt verwendet werden Mikroorganismenstämme der Art Escherichia coli, die einen deregulierten Cystein-Stoffwechsel besitzen.

Bevorzugt handelt es dabei sich um Escherichia coli-Stämme wie beispielsweise in WO 97/15673 oder in EP 0885962 A1 (entspricht der US Anmeldung mit der Serial Number SN 09/097759) oder in DE 19949579 beschreiben. Nach den in diesen Patentanmeldungen beschriebenen Verfahren kann in beliebigen Stämmen durch Transformation mit einem Plasmid, das z.B. ein feedbackresistentes cysE-Allel und/oder ein Efflux-Gen trägt, der Cystein-Stoffwechsel dereguliert werden.

Das erfindungsgemäße Verfahren zur Herstellung der nicht-proteinogenen L-Aminosäuren mit Hilfe eines Mikroorganismenstammes wird in einem Fermenter in an und für sich bekannter Art und Weise aber unter zusätzlicher Zugabe einer nukleophilen Verbindung durchgeführt.

Die Anzucht des Mikroorganismenstammes im Fermenter erfolgt als kontinuierliche Kultur, als batch-Kultur oder vorzugsweise als fed-batch-Kultur. Besonders bevorzugt werden eine C-Quelle und eine nukleophile Verbindung während der Fermentation kontinuierlich zudosiert.

Die Dosierung der nukleophilen Verbindung beginnt nach dem Animpfen oder vorzugsweise nach einer Anwachsphase. Besonders bevorzugt beginnt die Dosierung 6-8 Stunden nach dem Beginn der Fermentation und dauert bis zum Ende der Fermentation.

Die Menge an zugegebener nukleophiler Verbindung hängt von ihrer Toxizität für den Mikroorganismus ab und bewegt sich im Bereich von 10 bis 1000 mmol pro Liter Anfangsvolumen des Fermentationsmediums. Besonders bevorzugt ist eine Dosierung von 50 bis 500 mmol pro Liter Anfangsvolumen des Fermentationsmediums.

Als C-Quellen für die Fermentation dienen vorzugsweise Zucker, Zuckeralkohole oder organische Säuren. Besonders bevorzugt werden im erfindungsgemäßen Verfahren als C-Quellen Glukose, Laktose oder Glycerin eingesetzt.

Bevorzugt ist die Dosierung von Glukose in einer Form, die gewährleistet, dass der Gehalt im Fermenter während der Fermentation in einem Bereich von 0,1 - 50 g/l gehalten wird. Besonders bevorzugt ist ein Bereich von 0,5 - 10 g/l.

Als N-Quelle werden im erfindungsgemäßen Verfahren vorzugsweise Ammoniak, Ammoniumsalze oder Proteinhydrolysate verwendet.

Als weitere Medienzusätze können Salze der Elemente Phosphor, Schwefel, Chlor, Natrium, Magnesium, Stickstoff, Kalium, Calcium, Eisen und in Spuren (d.h. in µM Konzentrationen) Salze der Elemente Molybdän, Bor, Kobalt, Mangan, Zink und Nickel zugesetzt werden.

Desweiteren können organische Säuren (z.B. Acetat, Citrat), Aminosäuren (z.B. Isoleucin) und Vitamine (z.B. B1, B6) dem Medium zugesetzt werden.

Als komplexe Nährstoffquellen können z.B. Hefeextrakt, Maisquellwasser, Sojamehl oder Malzextrakt zum Einsatz kommen.

Der pH-Wert des Fermenationsmediums liegt im Bereich von 4-10. Bevorzugt ist ein Bereich von 6-8. Besonders bevorzugt ist ein pH-Bereich von 6,5 bis 7,5

Die Inkubationstemperatur beträgt 15 - 45 °C. Bevorzugt ist eine Temperatur zwischen 30 - 37 °C.

Die Fermentation wird vorzugsweise unter aeroben Wachstumsbedingungen durchgeführt. Der Sauerstoffeintrag in den-Fermenter erfolgt mit Pressluft oder mit reinem Sauerstoff.

Mikroorganismen, die nach dem beschriebenen Verfahren fermentiert werden, sezernieren in einer Fermentationszeit von 1 bis 4 Tage die entsprechenden nicht-proteinogenen L-Aminosäuren mit hoher Effizienz in das Kulturmedium.

Bei Zufuhr einer nukleophilen Substanz sezernieren Mikroorganismen mit dereguliertem Cystein-Stoffwechsel während der Fermentation nicht-proteinogene Aminosäuren der allgemeinen Formel (6) in L-Konfiguration: 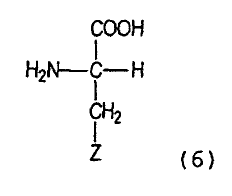 wobei Z ein einwertiger Rest ausgewählt aus den Formeln (7) bis (13) ist 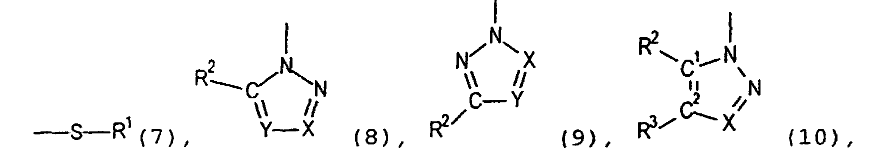 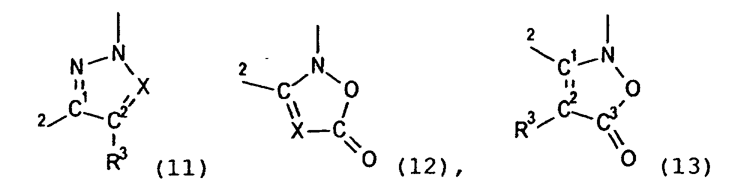 sowie deren Ester, Ether oder Salze ist,
und R¹ ,R², R³, R⁴, X und Y die bereits zu den Formeln (1) bis (5) genannte Bedeutung haben.

Das erfindungsgemäßen Verfahren ermöglicht es erstmals 1,2,3,4-Tetrazol-l-yl-L-alanin (14) bzw. 1,2,3,4-Tetrazol-2-yl-L-alanin (15) einschließlich deren Ester, Ether oder Salze, sowie um 1,2,3-Triazol-1-yl-L-alanin (16) bzw. 1,2,3-Triazol-2-yl-L-alanin (17) einschließlich deren Ester, Ether oder Salze, 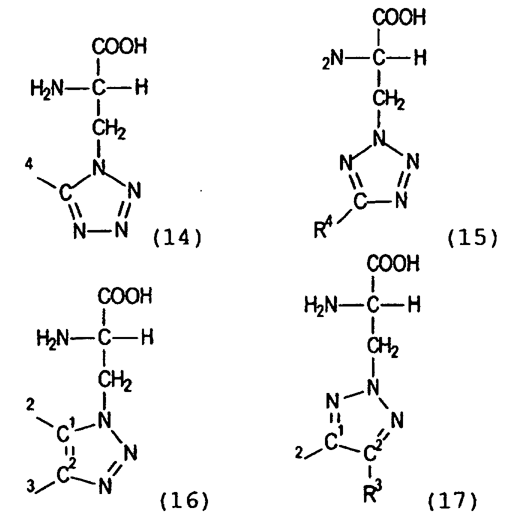 wobei R¹ ,R², R³ und R⁴ die bereits zu den Formeln (1) bis (5) genannte Bedeutung haben, herzustellen.

Das erfindungsgemäße Verfahren ermöglicht es außerdem, erstmals S-Heteroaryl-L-cysteine der Formel (18) 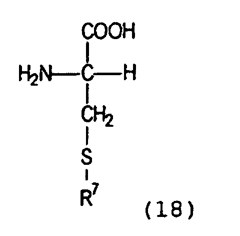 herzustellen, wobei R⁷ folgende Bedeutung hat:
Pyrrolyl, Pyrazolyl, Thiazolyl, Oxazolyl, Furanyl, Pyridinyl, Pyrazinyl, Benzimidazolyl, Benzotriazolyl, Purinyl.
Die Erfindung betrifft daher die genannten Verbindungen.
Besonders bevorzugte Verbindungen sind:
   - 1,2,3-Benzotriazol-1-yl-L-alanin (R² und R³ sind gleich und bedeuten [-CR⁵=CR⁶-], wobei R⁵ und R⁶ gleich H sind und R² und R³ zu einem aromatischen Ring verknüpft sind),
   - 1,2,3-Benzotriazol-2-yl-L-alanin (R² und R³ sind gleich und bedeuten [-CR⁵=CR⁶-], wobei R⁵ und R⁶ gleich H sind und R² und R³ zu einem aromatischen Ring verknüpft sind),
   - 5-Carboxy-1,2,3-benzotriazol-1-yl-L-alanin (R² und R³ sind verschieden und bedeuten [-CR⁵=CR⁶-], wobei R⁵ und R⁶ im Falle von R³ gleich H sind und im Falle von R² R⁵ gleich H und R⁶ gleich -COOH ist, und R² und R³ zu einem aromatischen Ring verknüpft sind)
   - 5-Carboxy-1,2,3-benzotriazol-2-yl-L-alanin (R² und R³ sind verschieden und bedeuten [-CR⁵=CR⁶-], wobei R⁵ und R⁶ im Falle von R³ gleich H sind und im Falle von R² R⁵ gleich H und R⁶ gleich -COOH ist, und R² und R³ zu einem aromatischen Ring verknüpft sind)
   - Thiazol-2-yl-L-cystein
   - Pyridin-2-yl-L-cystein.

Vorzugsweise wird das Produkt nach Abtrennen der Biomasse mittels bekannter Methoden (z.B. Filtration, Zentrifugation) aus dem Kulturüberstand isoliert. Solche Methoden zur Isolierung von Aminosäuren sind dem Fachmann ebenfalls bekannt. Sie umfassen z.B. Extraktion, Adsorption, Ionenaustauscher-Chromatographie, Präzipitation, Kristallisation.

Die folgenden Beispiele dienen der weiteren Erläuterung der Erfindung. Der Bakterienstamm *Escherichia coli* W3110 / pA-CYC184-cysEX-GAPDH-ORF306, der für die Ausführung der Beispiele verwendet wurde, wurde bei der DSMZ (Deutsche Sammlung für Mikroorganismen und Zellkulturen GmbH, D-38142 Braunschweig) unter der Nummer DSM 13495 gemäß Budapester Vertrag hinterlegt.

### Beispiel 1: Vorkultur des Produktionsstammes

Als Vorkultur für die Fermentation wurden 20 ml LB-Medium (10 g/l Trypton, 5 g/l Hefeextrakt, 10 g/l NaCl), das zusätzlich 15 mg/l Tetracyclin enthielt, mit dem Stamm W3110 / pACYC184-cysEX-GAPDH-ORF306 (beschrieben in EP 0885962 A1, beimpft und bei 30 °C und 150 rpm in einem Schüttler inkubiert. Nach sieben Stunden wurde der gesamte Ansatz in 100 ml SM1-Medium (12 g/l K₂HPO₄; 3 g/l KH₂PO₄; 5 g/l (NH₄)₂SO₄; 0,3 g/l MgSO₄ x 7 H₂O; 0,015 g/l CaCl₂ x 2 H₂O; 0,002 g/l FeSO₄ x 7 H₂O; 1 g/l Na₃Citrat x 2 H₂O; 0,1 g/l NaCl; 1 ml/l Spurenelementlösung bestehend aus 0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O), das mit 5 g/l Glukose; 0,5 mg/l Vitamin B₁ und 15 mg/l Tetracyclin supplementiert wurde, überführt. Die weitere Inkubation erfolgte bei 30 °C für 17 Stunden bei 150 rpm.

### Beispiel 2: Fermentative Herstellung von S-[2,3-Dihydroxy-4-mercaptobutyl]-L-cystein

Als Fermenter diente ein Biostat M-Gerät der Firma Braun Biotech (Melsungen, D) mit einem maximalen Kulturvolumen von 2 1. Mit der in Beispiel 1 beschriebenen Vorkultur (optische Dichte bei 600 nm von ca. 3) wurde der Fermenter mit 900 ml Fermentationsmedium (15 g/l Glukose; 10 g/l Trypton; 5 g/l Hefeextrakt; 5 g/l (NH₄)₂SO₄; 1,5 g/l KH₂PO₄; 0,5 g/l NaCl; 0,3 g/l MgSO₄ x 7 H₂O; 0,015 g/l CaCl₂ x 2 H₂O; 0,075 g/l FeSO₄ x 7 H₂O; 1 g/l Na₃Citrat x 2 H₂O und 1 ml/l Spurenelementlösung s.o., 5 mg/l Vitamin B1 und 15 mg/l Tetracyclin, eingestellt auf pH 7,0 mit 25 % Ammoniak) beimpft. Während der Fermentation wurde eine Temperatur von 32 °C eingestellt und der pH-Wert durch Zudosierung von 25 % Ammoniak bei einem Wert von 7,0 konstant gehalten. Die Kultur wurde mit entkeimter Druckluft bei 1,5 vol/vol/min begast und mit einer Rührerdrehzahl von 200 rpm gerührt. Nach Absinken der Sauerstoffsättigung auf einen Wert von 50 % wurde die Drehzahl über ein Kontrollgerät bis zu einem Wert von 1200 rpm erhöht, um 50 % Sauerstoffsättigung zu erhalten (Bestimmt mit einer pO2-Sonde, kalibriert auf 100% Sättigung bei 900 rpm).

Nach acht Stunden erfolgte eine Zudosierung einer 1 M Dithiothreitol-Lösung mit einer Rate von 2 mmol/h. Glukose wurde aus einer 56 % Stammlösung zugefüttert, sobald der Gehalt im Fermenter von anfänglich 15 g/l auf ca. 5-10 g/l abgesunken war. Die Glukose-Fütterung erfolgte mit einer Flußrate von 8-14 ml/h, wobei die Glukosekonzentration zwischen 0,5 - 10 g/l konstant gehalten wurde. Die Glukose-Bestimmung wurde mit dem Glukoseanalysator der Firma YSI (Yellow Springs, Ohio, USA) durchgeführt.
Die Fermentationsdauer betrug 48 Stunden. Nach dieser Zeit wurden Proben entnommen und die Zellen durch Zentrifugation vom Kulturmedium abgetrennt. Die resultierenden Kulturüberstände wurden durch reversed phase HPLC an einer LUNA 5 µ C18(2)-Säule (Phenomenex, Aschaffenburg, Deutschland) aufgetrennt. Als Eluent diente verdünnte Phosphorsäure (0,1 ml konz. Phosphorsäure / 1) bei einer Flußrate von 0,5 ml/min. S-Mercaptodihydroxybutyl-L-cystein wird bei einer Retentionszeit von 86,7 min eluiert. Die Ausbeute betrug 2,5 g/l.

### Beispiel 3: Fermentative Herstellung von S-Phenyl-L-cystein

Die Bakterien wurden wie in Beispiel 1 und 2 beschrieben kultiviert. Nach acht Stunden erfolgte eine Zudosierung einer 1 M Na-Thiophenol-Suspension mit einer Rate von 2 mmol/h.
S-Phenyl-L-cystein wird mit der in Beispiel 2 beschriebenen HPLC-Methode bei einer Retentionszeit von 88 min eluiert. Die Ausbeute betrug 2,1 g/l.

### Beispiel 4: Fermentative Herstellung von 1,2-Pyrazolyl-L-alanin

Die Bakterien wurden wie in Beispiel 1 und 2 beschrieben kultiviert. Nach acht Stunden erfolgte eine Zudosierung einer 1 M 1,2-Pyrazol-Lösung mit einer Rate von 4 mmol/h. 1,2-Pyrazolyl-L-alanin wird mit der in Beispiel 2 beschriebenen HPLC-Methode bei einer Retentionszeit von 8,4 min eluiert. Die Ausbeute betrug 6,1 g/l.

### Beispiel 5: Fermentative Herstellung von 1,2,4-Triazolyl-Lalanin

Die Bakterien wurden wie in Beispiel 1 und 2 beschrieben kultiviert. Nach acht Stunden erfolgte eine Zudosierung einer 1 M 1,2,4-Triazol-Lösung mit einer Rate von 4 mmol/h. 1,2,4-Triazol-1-yl-L-alanin wird mit der in Beispiel 2 beschriebenen HPLC-Methode bei einer Retentionszeit von 5,8 min eluiert. Die Ausbeute betrug 4,6 g/l.

### Beispiel 6: Fermentative Herstellung von 1,2,3,4-Tetrazolyl-Lalanin

Die Bakterien wurden wie in Beispiel 1 und 2 beschrieben kultiviert. Nach acht Stunden erfolgte eine Zudosierung einer 1 M 1,2,3,4-Tetrazol-Lösung mit einer Rate von 4 mmol/h. Bei der Fermentation entstehen die beiden Isomere 1,2,3,4-Tetrazol-1-yl-L-alanin und 1,2,3,4-Tetrazol-2-yl-L-alanin. Diese werden mit der in Beispiel 2 beschriebenen HPLC-Methode bei einer Retentionszeit von 5,4 bzw. 5,7 min eluiert. Die Ausbeute betrug als Summe der beiden Isomere 3,9 g/l.

### Beispiel 7: Fermentative Herstellung von 5-Carboxy-1,2,3-benzotriazolyl-L-alanin

Die Bakterien wurden wie in Beispiel 1 und 2 beschrieben kultiviert. Nach acht Stunden erfolgte eine Zudosierung einer Suspension von 1 M 1,2,3-Benzotriazol-5-carbonsäure in 0,5 M NaOH mit einer Rate von 4 mmol/h.
Bei der Fermentation entstehen alle drei Isomere 5-Carboxy-1,2,3-benzotriazol-1-yl-L-alanin, 5-Carboxy-1,2,3-benzotriazol-2-yl-L-alanin und 5-Carboxy-1,2,3-benzotriazol-3-yl-L-alanin, wobei aber das Hauptprodukt 5-Carboxy-1,2,3-benzotriazol-2-yl-L-alanin darstellt. Dieses wird mit der in Beispiel 2 beschriebenen HPLC-Methode bei einer Retentionszeit von 67,5 min eluiert. Die Ausbeute betrug 5,2 g/l.

### Beispiel 8: Fermentative Herstellung von 1,2,4-Oxadiazolidin-2,5-dionyl-L-alanin (= Quisqualinsäure)

Die Bakterien wurden wie in Beispiel 1 und 2 beschrieben kultiviert. Nach acht Stunden erfolgte eine Zudosierung einer 2 M Lösung von 1,2,4-Oxadiazolidin-2,5-dion in Dimethylsulfoxid mit einer Rate von 2 mmol/h.
Quisqualinsäure wird mit der in Beispiel 2 beschriebenen HPLC-Methode bei einer Retentionszeit von 5,6 min eluiert. Die Ausbeute betrug 2,2 g/l.

### Bespiel 9: Isolierung von 1,2-Pyrazolyl-L-alanin aus Fermenterbrühe

Zunächst wurden durch Zentrifugation von 0,6 1 Fermenterbrühe bei 5 000 g die Zellen abgetrennt. Der Überstand wurde zur Entfärbung mit 10 g Aktivkohle versetzt, 2 h bei Raumtemperatur gerührt und anschließend filtriert. Die erhaltene Lösung wurde mit 2 M NaOH auf einen pH-Wert von 6,0 eingestellt, auf eine KatIonenaustauschersäule Amberjet 1200 / H+ (Rohm and Haas S.A., Chauny, France; 250 ml Gelbett) aufgetragen und gebundene Substanzen mit 1 M NaCl eluiert. Die Elutionsfraktionen wurden vereinigt (500 ml), mit 2 M NaOH auf einen pH-Wert von 6,0 eingestellt und auf 100 ml eingeengt. Die Probe wurde für 16 h bei 4 °C gelagert. Das resultierende Kristallisat wurde durch Filtation gewonnen, mit 50 ml Ethanol gewaschen und anschließend getrocknet.

### Beispiel 10: Fermentative Herstellung von S-Thiazol-2-yl-Lcystein

Die Bakterien wurden wie in Beispiel 1 und 2 beschrieben kultiviert. Nach acht Stunden erfolgte eine Zudosierung einer 1 M 2-Mercaptothiazol-Lösung mit einer Rate von 2 mmol/h. S-Thiazol-2-yl-L-cystein wird mit der in Beispiel 2 beschriebenen HPLC-Methode bei einer Retentionszeit von 33,7 min eluiert. Die Ausbeute betrug 6,1 g/l.

### Beispiel 11: Fermentative Herstellung von S-1,2,4-Triazol-3-yl-L-cystein

Die Bakterien wurden wie in Beispiel 1 und 2 beschrieben kultiviert. Nach acht Stunden erfolgte eine Zudosierung einer 1 M 3-Mercaptotriazol-Lösung mit einer Rate von 2 mmol/h. S-Thiazol-2-yl-L-cystein wird mit der in Beispiel 2 beschriebenen HPLC-Methode bei einer Retentionszeit von 8,2 min eluiert. Die Ausbeute betrug 5,5 g/l.

## Patentansprüche

1. Verfahren zur Herstellung von nicht-proteinogenen L-Aminosäuren durch direkte Fermentation eines Mikroorganismenstammes mit einem modifizierten cys-E-Allel, **dadurch gekennzeichnet, daß** während der Fermentation eine nukleophile Verbindung in derartigen Mengen dem Fermentationsansatz zudosiert wird, daß diese zur Produktion von nicht-proteinogenen L-Aminosäuren durch den Mikroorganismenstamm führt.

2. Verfahren nach Anspruch 1, **dadurch gekennzeichnet, daß** am Ende der Fermentation die nicht-proteinogene L-Aminosäuren aus dem Fermentationsansatz mittels an sich bekannter Methoden abgetrennt wird.

3. Verfahren nach Anspruch 1 oder 2, **dadurch gekennzeichnet, daß** eine nukleophile Verbindung die einen Rest ausgewählt aus der Gruppe 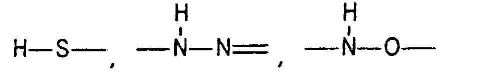 umfasst, zudosiert wird.

4. Verfahren nach Anspruch 1 oder 2, **dadurch gekennzeichnet, daß** eine nukleophile Verbindung ausgewählt aus der folgenden Gruppe zugegeben wird:
- Thiol der allgemeinen Formel (1):
H―S―R₁ (1)
wobei R¹ einwertiger substituierter oder nicht substituierter Alkyl-, Alkoxy-, Aryl- oder Heteroarylrest mit maximal 15 C-Atomen bedeutet;
- Azol der allgemeinen Formel (2) oder (3): 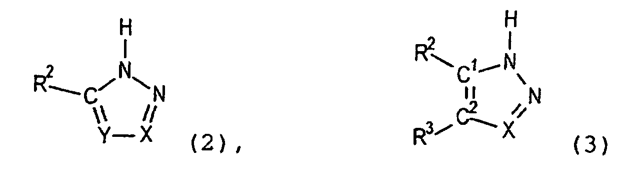 sowie deren Ester, Ether oder Salze,
wobei X und Y gleich oder verschieden sind und CR⁴ oder N bedeuten und R⁴ -H, -COOH, -OH, -NH₂, -NO₂⁻, -SH, -SO₃⁻, -F, -Cl, -Br, -I, C₁-C₅-Alkylcarbonyl- oder R¹ bedeutet und R¹ die zu Formel (1) genannte Bedeutung hat und
wobei R² und R³ gleich oder verschieden sind und R⁴ bedeuten oder wobei C¹ und C² in Formel (3) anstelle der Substituenten R² und R³ mittels einer Brücke [-CR⁵R⁶-]ₐ mit a gleich 1,2, 3 oder 4 zu einem Ring verknüpft sind, wobei
R⁵ und R⁶ gleich oder verschieden sind und R⁴ bedeuten und eine oder mehrere nicht benachbarte Gruppen [-CR⁵R⁶-] durch Sauerstoff, Schwefel oder einen ggf. mit C₁-C₅-Alkyl- substituierten Iminorest ersetzt sein können und
zwei benachbarte Gruppen [-CR⁵R⁶-] durch eine Gruppe [-CR⁵=CR⁶-] oder durch eine Gruppe [-CR⁵=N-] ersetzt sein können.
- Isoxazolinon der allgemeinen Formel (4) oder (5): 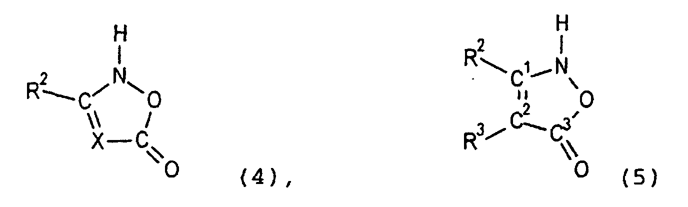 sowie deren Ester, Ether oder Salze,
wobei X, R¹ , R² und R³ die bereits genannte Bedeutung haben und
wobei C¹ und C² in Formel (5) anstelle der Substituenten R² und R³ mittels einer wie für Formel (3) definierten Brücke zu einem Ring verknüpft sein können.

5. Verfahren nach einem der Ansprüche 1 bis 4, **dadurch gekennzeichnet, daß** ein Mikroorganismenstamm ausgewählt aus der Gruppe der folgenden Stämme im erfindungsgemäßen Verfahren eingesetzt wird: Stämme mit modifizierten cysE-Allelen; Stämme, die Efflux-Gene enthalten; Stämme mit einer geänderten CysB-Aktivität; Stämme, die unter Einsatz unspezifischer Mutagenese-Methoden kombiniert mit Screening-Methoden für Cystein-Überproduktion oder verminderten Cystein-Abbau gewonnen werden.

6. Verfahren nach einem der Ansprüche 1 bis 5, **dadurch gekennzeichnet, daß** als Mikroorganismenstamm ein Escherichia coli-Stamm eingesetzt wird.

7. Verfahren nach Anspruche 1, 5 oder 6, **dadurch gekennzeichnet, daß** die Anzucht des Mikroorganismenstammes im Fermenter als kontinuierliche Kultur, als batch-Kultur oder als fed-batch-Kultur erfolgt.

8. Verfahren nach einem der Ansprüche 1 bis 7, **dadurch gekennzeichnet, daß** eine C-Quelle und eine nukleophile Verbindung während der Fermentation kontinuierlich zudosiert werden.

9. Verfahren nach einem der Ansprüche 1 bis 8, **dadurch gekennzeichnet, daß** die Dosierung der nukleophilen Verbindung 6-8 Stunden nach dem Beginn der Fermentation beginnt und bis zum Ende der Fermentation dauert.

10. Verfahren nach einem der Ansprüche 1 bis 9, **dadurch gekennzeichnet, daß** die Menge an zugegebener nukleophiler Verbindung sich im Bereich von 10 bis 1000 mmol pro Liter Anfangsvolumen des Fermentationsmediums bewegt.

11. Verfahren nach einem der Ansprüche 1 bis 10, **dadurch gekennzeichnet, daß** als C-Quellen Zucker, Zuckeralkohole oder organische Säuren eingesetzt werden.

12. Verfahren nach Anspruch 11, **dadurch gekennzeichnet, daß** die Dosierung der C-Quelle in einer Form erfolgt, die gewährleistet, dass der Glukose-Gehalt im Fermenter in einem Bereich von 0,1 - 50 g/l gehalten wird

13. Verfahren nach einem der Ansprüche 1 bis 12, **dadurch gekennzeichnet, daß** als N-Quelle Ammoniak, Ammoniumsalze oder Proteinhydrolysate eingesetzt werden.

14. Verfahren nach einem der Ansprüche 1 bis 13, **dadurch gekennzeichnet, daß** der pH-Wert des Fermenationsmediums im Bereich von 4-10 liegt und die Inkubationstemperatur 15 - 45 °C beträgt.

15. Verfahren nach einem der Ansprüche 1 bis 14, **dadurch gekennzeichnet, daß** es unter aeroben Wachstumsbedingungen durchgeführt wird.

16. Verfahren nach einem der Ansprüche 1 bis 15, **dadurch gekennzeichnet, daß** die nicht-proteinogene L-Aminosäure nach Abtrennen der Biomasse aus dem Fermetationsansatz mittels Filtration oder Zentrifugation aus dem Kulturüberstand mittels Extraktion, Adsorption, Ionenaustauscher-Chromatographie, Präzipitation, oder Kristallisation isoliert wird.

17. Verfahren nach einem der Ansprüche 1 bis 16 **dadurch gekennzeichnet, daß** es sich bei der nicht-proteinogenen L-Aminosäure um eine Aminosäure der allgemeinen Formel (6) in L-Konfiguration: 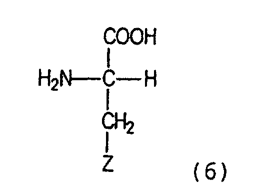 handelt, wobei Z ein einwertiger Rest ausgewählt aus den Formeln (7) bis (13) ist 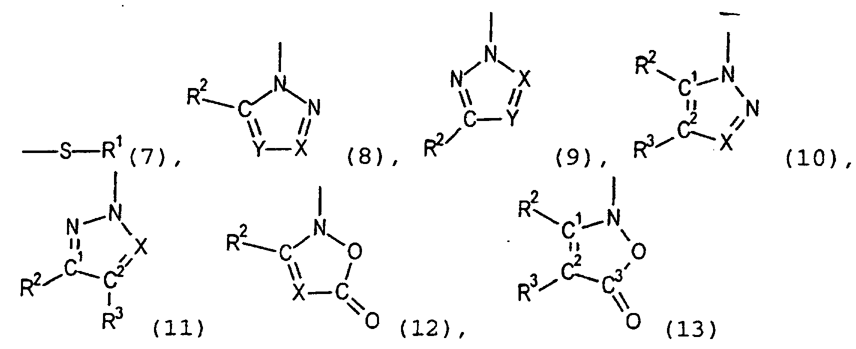 sowie deren Ester, Ether oder Salze ist,
und R¹ ,R², R³, R⁴, X und Y die bereits zu den Formeln (1) bis (5) genannte Bedeutung haben.

18. Verbindung ausgewählt aus der Gruppe 1,2,3,4-Tetrazol-1-yl-L-alanin (14), 1,2,3,4-Tetrazol-2-yl-L-alanin (15), 1,2,3-Triazol-1-yl-L-alanin (16) 1,2,3-Triazol-2-yl-L-alanin (17) einschließlich deren Ester, Ether oder Salze, 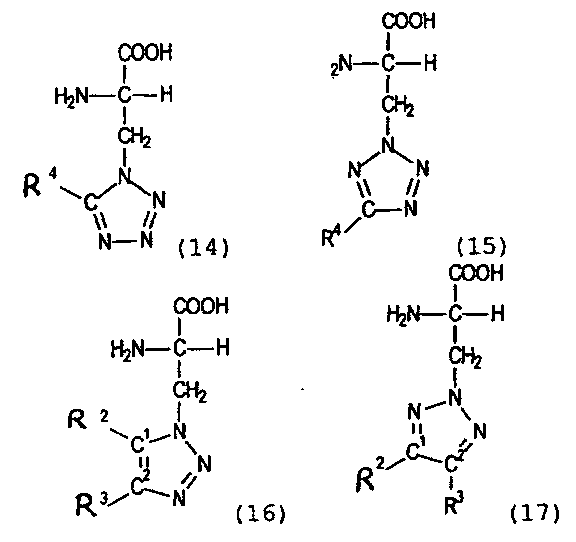 wobei R¹ ,R², R³ und R⁴ die bereits in Anspruch 4 genannte Bedeutung haben.

19. S-Heteroaryl-L-cystein-Derivat der Formel 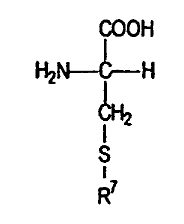 wobei R⁷ folgende Bedeutung hat: Pyrrolyl-, Pyrazolyl-, Tetrazolyl-, Thiazolyl-, Oxazolyl-, Furanyl-, Pyridinyl-, Pyrazinyl-, Benzimidazoyl-, Benzotriazolyl- und Purinyl-Rest

## Claims

1. Process for producing non-proteinogenic L-amino acids by direct fermentation of a microorganism strain having a modified cysE allele, **characterized in that**, during the fermentation, a nucleophilic compound is added to the fermentation batch in amounts such that this leads to the production of non-proteinogenic L-amino acids by the microorganism strain.

2. Process according to Claim 1, **characterized in that**, at the end of the fermentation, the non-proteinogenic L-amino acids are removed from the fermentation batch by methods known per se.

3. Process according to Claim 1 or 2, **characterized in that** a nucleophilic compound is added which has a radical selected from the group consisting of

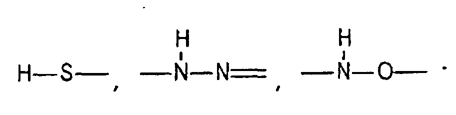

4. Process according to Claim 1 or 2, **characterized in that** a nucleophilic compound is added selected from the following group consisting of:
- Thiol of the general formula (1):
H―S―R₁ (1)
where R¹ is monovalent substituted or unsubstituted alkyl, alkoxy, aryl or heteroaryl radical having a maximum of 15 carbon atoms;
- azole of the general formula (2) or (3): 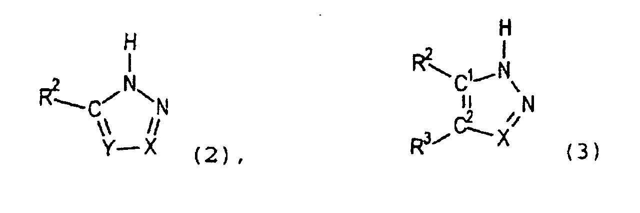 and their esters, ethers or salts,
where X and Y are identical or different and denote CR⁴ or N, and R⁴ is -H, -COOH, -OH, -NH₂, -NO₂⁻, -SH, -SO₃⁻, -F, -Cl, -Br, -I, C₁-C₅-alkylcarbonyl- or R¹, and R¹ has the meaning specified under formula (1) and
where R² and R³ are identical or different and are R⁴
or where C¹ and C² in formula (3), instead of the substituents R² and R³, are linked by means of a bridge [-CR⁵R⁶-]ₐ, where a is 1, 2, 3 or 4, to form a ring, where
R⁵ and R⁶ are identical or different and are R⁴ and
one or more non-adjacent groups [-CR⁵R⁶-] can be replaced by oxygen, sulphur, or an imino radical, which may be unsubstituted or substituted by C₁-C₅-alkyl, and
two adjacent groups [-CR⁵R⁶-] can be replaced by a group [-CR⁵=R⁶-] or by a group [-CR⁵=N-];
- Isoxazolinone of the general formula (4) or (5) : 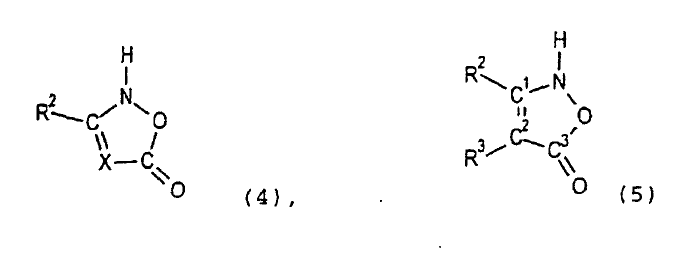 and their esters, ethers or salts,
where X, R¹, R², R³ have the meaning specified above
and
where C¹ and C² in formula (5), instead of the substituents R² and R³, can be linked by means of a bridge defined as for formula (3) to form a ring.

5. Process according to one of Claims 1 to 4, **characterized in that** a microorganism strain is used in the inventive process which is selected from the group consisting of the following strains: strains having modified cysE alleles; strains that contain efflux genes; strains having modified CysB activity; strains which are produced using nonspecific mutagenesis methods combined with screening methods for cysteine overproduction or reduced cysteine degradation.

6. Process according to one of Claims 1 to 5, **characterized in that** the microorganism strain used is an Escherichia coli strain.

7. Process according to Claims 1, 5 or 6, **characterized in that** the microorganism strain is grown in the fermenter as a continuous culture, as batch culture or as fed-batch culture.

8. Process according to one of Claims 1 to 7, **characterized in that** a carbon source and a nucleophilic compound are added continuously during the fermentation.

9. Process according to one of Claims 1 to 8, **characterized in that** addition of the nucleophilic compound begins 6-8 hours after the start of fermentation and lasts until the end of fermentation.

10. Process according to one of Claims 1 to 9, **characterized in that** the amount of nucleophilic compound to be added is in the range from 10 to 1 000 mmol per litre of initial volume of fermentation medium.

11. Process according to one of Claims 1 to 10, **characterized in that** the carbon sources used are sugars, sugar alcohols or organic acids.

12. Process according to Claim 11, **characterized in that** the carbon source is added in a form which ensures that the glucose content in the fermenter is kept in a range of 0.1 - 50 g/l.

13. Process according to one of Claims 1 to 12, **characterized in that** the nitrogen source used is ammonia, ammonium salts or protein hydrolysates.

14. Process according to one of Claims 1 to 13, **characterized in that** the pH of the fermentation medium is in the range of 4-10 and the incubation temperature is 15 - 45°C.

15. Process according to one of Claims 1 to 14, **characterized in that** it is carried out under aerobic growth conditions.

16. Process according to one of Claims 1 to 15, **characterized in that** the non-proteinogenic L-amino acid, after removing the biomass from the fermentation batch by filtration or centrifugation, is isolated from the culture supernatant by means of extraction, adsorption, ion-exchange chromatography, precipitation or crystallization.

17. Process according to one of Claims 1 to 16, **characterized in that** the non-proteinogenic L-amino acid is an amino acid of the general formula (6) in the L configuration: 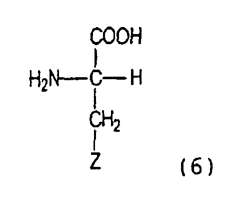 where Z is a monovalent radical selected from the formulae (7) to (13): 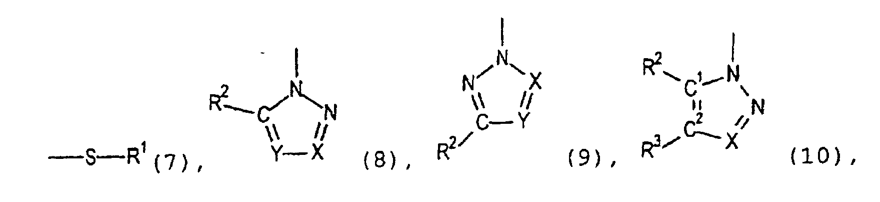 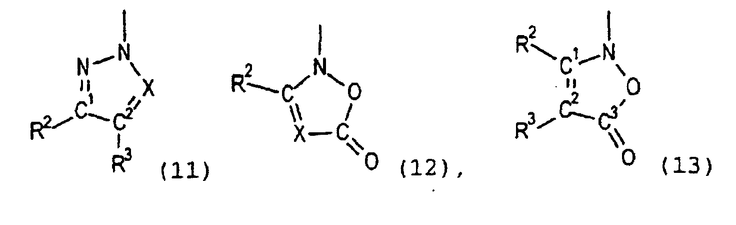 and their esters, ethers or salts,
and R¹, R², R³, R⁴, X and Y have the meaning specified for the formulae (1) to (5).

18. Compound selected from the group consisting of 1,2,3,4-tetrazol-1-yl-L-alanine (14), 1,2,3,4-tetrazol-2-yl-L-alanine (15), 1,2,3-triazol-1-yl-L-alanine (16), 1,2,3-triazol-2-yl-L-alanine (17) including their esters, ethers or salts, 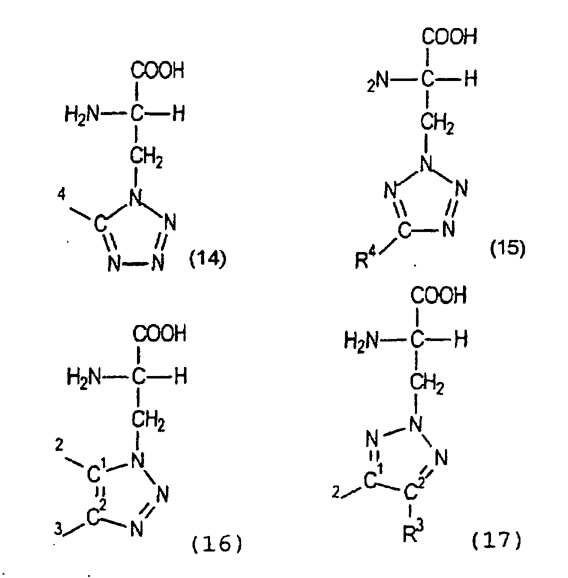 where R¹, R², R³ and R⁴ have the meaning specified in Claim 4.

19. S-Heteroaryl-L-cysteine derivative of the formula 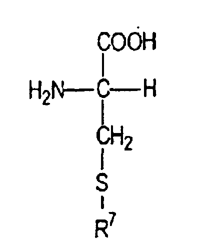 where R⁷ has the following meaning: pyrrolyl, pyrazolyl, tetrazolyl, thiazolyl, oxazolyl, furanyl, pyridinyl, pyrazinyl, benzimidazoyl, benzotriazolyl and purinyl radical.

## Revendications

1. Procédé pour la production de L-aminoacides non protéinogènes par fermentation directe d'une souche de microorganismes avec un allèle cys-E modifié, **caractérisé en ce qu'**on ajoute par dosage pendant la fermentation, à la charge .de fermentation, un composé nucléophile en des quantités telles que celui-ci conduit à la production de L-aminoacides non protéinogènes par la souche de microorganismes.

2. Procédé selon la revendication 1, **caractérisé en ce qu'**à la fin de la fermentation, les L-aminoacides non protéinogènes sont séparés de la charge de fermentation au moyen de procédés connus en soi.

3. Procédé selon la revendication 1 ou 2, **caractérisé en ce qu'**on ajoute par dosage un composé nucléophile présentant un radical choisi dans le groupe:

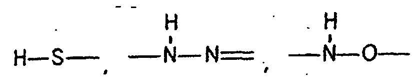

4. Procédé selon la revendication 1 ou 2, **caractérisé en ce qu'**on ajoute un composé nucléophile choisi dans le groupe suivant :
- thiol de formule générale (1):
H-S-R₁ (1)
R¹ signifiant un radical alkyle, alcoxy, aryle ou hétéroaryle monovalent, substitué ou non substitué comprenant au maximum 15 atomes de carbone ;
- azole de formule générale (2) ou (3) : 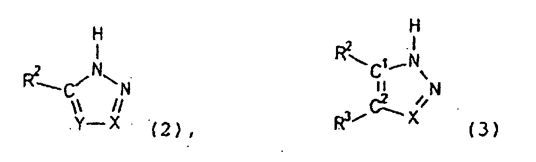 ainsi que leurs esters, éthers ou sels,
X et Y étant identiques ou différents et signifiant CR⁴ ou N et R⁴ signifiant -H, -COOH, -OH, -NH₂, -NO₂⁻, -SH, -SO₃⁻, -F, -Cl, -Br, -I, (alkyle en C₁ à C₅)carbonyle ou R¹ et R¹ ayant la signification mentionnée pour la formule (1) et R² et R³ étant identiques ou différents et signifiant R⁴
ou C¹ et C² dans la formule (3), au lieu des substituants R² et R³, étant reliés en formant un cycle au moyen d'un pont [-CR⁵R⁶-]ₐ, a valant 1, 2, 3 ou 4,
R⁵ et R⁶ étant identiques ou différents et signifiant R⁴ et un ou plusieurs groupements [-CR⁵R⁶-] non adjacents pouvant être remplacés par oxygène, soufre ou un radical imino éventuellement substitué par un groupement alkyle en C₁ à C₅, et deux groupements adjacents [-CR⁵R⁶-] pouvant être remplacés par un groupement [-CR⁵=CR⁶-] ou par un groupement [-CR⁵=N-]
- isoxazolinone de formule générale (4) ou (5): 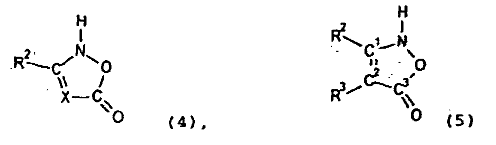 ainsi que leurs esters, éthers ou sels,
X, R¹, R² et R³ ayant la signification déjà mentionnée et
C¹ et C² dans la formule (5), au lieu des substituants R² et R³, pouvant être reliés en formant un cycle au moyen d'un pont tel que défini pour la formule (3).

5. Procédé selon l'une quelconque des revendications 1 à 4, **caractérisé en ce qu'**on utilise dans le procédé selon l'invention une souche de microorganismes choisie dans le groupe des souches suivantes : les souches avec des allèles cys-E modifiés ; les souches qui contiennent des gènes d'efflux ; les souches présentant une activité cysB modifiée ; les souches qui sont obtenues avec utilisation de procédés de mutagënèse non spécifiques, combinés avec des procédés de criblage orientés vers une surproduction de cystéine ou une dégradation moindre de la cystéine.

6. Procédé selon l'une quelconque des revendications 1 à 5, **caractérisé en ce qu'**on utilise comme souche de microorganismes une souche d'Escherichia coli.

7. Procédé selon les revendications 1, 5 ou 6, **caractérisé en ce que** la croissance de la souche de microorganismes est réalisée dans un fermenteur comme une culture continue, une culture par lots ou une culture semi-continue.

8. Procédé selon l'une quelconque des revendications 1 à 7, **caractérisé en ce qu'**on ajoute en dosant en continu une source de C et un composé nucléophile pendant la fermentation.

9. Procédé selon l'une quelconque des revendications 1 à 8, **caractérisé en ce que** le dosage du composé nucléophile commence 6 à 8 heures après le début de la fermentation et dure jusqu'à la fin de la fermentation.

10. Procédé selon l'une quelconque des revendications 1 à 9, **caractérisé en ce que** la quantité ajoutée de composé nucléophile se situe dans la plage de 10 à 1000 mmoles par litre de volume de départ du milieu de fermentation.

11. Procédé selon l'une quelconque des revendications 1 à 10, **caractérisé en ce qu'**on utilise comme sources de C du sucre, des alcools de sucre ou des acides organiques.

12. Procédé selon la revendication 11, **caractérisé en ce que** le dosage de la source de C est réalisé sous une forme qui garantit que la teneur en glucose dans le fermenteur est maintenue dans une plage de 0,1 à 50 g/l.

13. Procédé selon l'une quelconque des revendications 1 à 12, **caractérisé en ce qu'**on utilise comme sources de N de l'ammoniaque, des sels d'ammonium ou des hydrolysats de protéines.

14. Procédé selon l'une quelconque des revendications 1 à 13, **caractérisé en ce que** le pH du milieu de fermentation se situe dans la plage de 4 à 10 et la température d'incubation est de 15 à 45°C.

15. Procédé selon l'une quelconque des revendications 1 à 14, **caractérisé en ce qu'**il est réalisé sous des conditions de croissance aérobie.

16. Procédé selon l'une quelconque des revendications 1 à 15, **caractérisé en ce que** le L-aminoacide non protéinogène est isolé, après séparation de la biomasse de la charge de fermentation au moyen d'une filtration ou d'une centrifugation, du résidu de culture au moyen d'une extraction, d'une adsorption, d'une chromatographie par échange d'ions, d'une précipitation ou d'une cristallisation.

17. Procédé selon l'une quelconque des revendications 1 à 16, **caractérisé en ce qu'**il s'agit, pour le L-aminoacide non protéinogène, d'un aminoacide de formule générale (6) dans la configuration L: 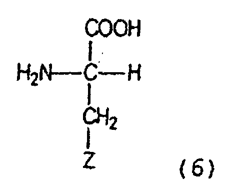 Z étant un radical monovalent choisi parmi les formules (7) à (13) 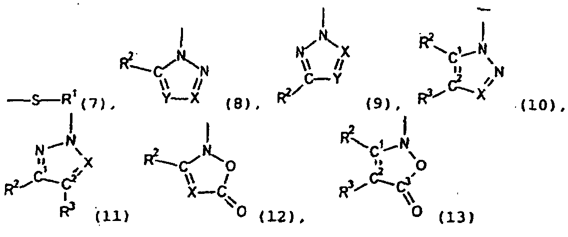 ainsi que leurs esters, éthers ou sels,
et R¹, R², R³, R⁴, X et Y ayant la signification déjà mentionnée pour les formules (1) à (5).

18. Composé choisi dans le groupe constitué par la 1,2,3,4-tétrazol-1-yl-L-alanine (14), la 1,2,3,4-tétrazol-2-yl-L-alanine (15), la 1,2,3-triazol-1-yl-L-alanine (16), la 1,2,3-triazol-2-yl-L-alanine (17), y compris leurs esters, éthers ou sels, 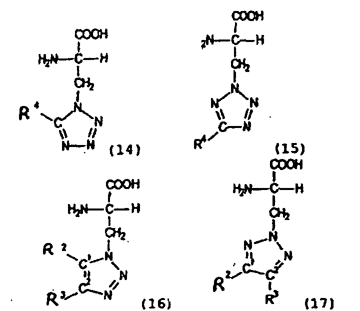 R¹, R², R³ et R⁴ ayant la signification déjà mentionnée dans la revendication 4.

19. Dérivé de la S-hétéroaryl-L-cystéine de formule 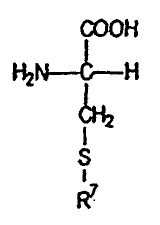 R⁷ ayant la signification suivante : radical pyrrolyle, pyrazolyle, tétrazolyle, thiazolyle, oxazolyle, furannyle, pyridinyle, pyrazinyle, benzimidazoyle, benzotriazolyle et purinyle.
